# EUROPEAN PATENT APPLICATION

(11) **EP 4 843 615 A1**
(43) Date of publication of application: **25.11.2026**
(21) Application number: 25177933.6
(22) Date of filing: 21.05.2025
(51) Int. Cl.: A61N 5/10, B65H 75/44

(54) **A CABLE ARRANGEMENT SYSTEM FOR A RADIOTHERAPY DEVICE WITH A ROTATABLE COLLIMATOR**

(71) Applicant: Varian Medical Systems, Inc., Palo Alto, CA 94304 (US)
(72) Inventor: Scollay, Stuart, Pacifica, 94044 (US); McWilliams, Christopher, SF, 94117 (US)
(74) Representative: Müller Schupfner & Partner Patent- und Rechtsanwaltspartnerschaft mbB (Muc)

(57) **Abstract**

The invention relates to a cable arrangement system for a radiotherapy device (10) with a rotatable collimator (1), the cable arrangement system comprising the rotatable collimator (1), and multiple supply cables (2) for the collimator (1), the cables having a sufficient length in order to compensate for the rotation of the collimator and being arranged in at least two groups (21, 22) of supply cables (2), each group (21, 22,) being arranged in such a way as to automatically adapt to a current rotational state of the collimator (1) via at least one variable compensation loop section (210, 220), the extent of the respective compensation loop section (210, 220) depending on the rotational state of the collimator (1), wherein the compensation loop sections (210, 220) of different groups (21, 22) are arranged separately from each other.

## Description

The invention relates to a cable arrangement system for a radiotherapy device with a rotatable collimator and a radiotherapy device.

*Independent of the grammatical term usage, individuals with male, female or other gender identities are included within the term.*

Medical radiation treatment devices used in oncology often include rotatable collimators that require precise control of motion and positioning. This is typically facilitated by cabling systems with cable windups to compensate for the rotational motion of the collimator. Typically, the collimator demands a robust cable management system that can accommodate multiple control and power cables while maintaining operational reliability. However conventional cable windups are challenging to integrate into such devices due to spatial constraints. Existing cable windup systems are typically constrained by the vertical clearance within the device. Such constraints lead to challenges in cable routing, excessive wear, and limited adaptability, making it difficult to meet performance requirements without compromising the device's compact design.

It is therefore an object of the invention to provide a cable arrangement system for a radiotherapy device with a rotatable collimator that allows a reliable cable management while improving on the problem of limited available space, in particular limited vertical space.

This object is met or exceeded by a cable arrangement system according to claim 1 and a radiotherapy device according to claim 15. Further advantages and features result from the dependent claims, the description and the attached figures.

According to a first aspect of the invention, a cable arrangement system for a radiotherapy device with a rotatable collimator is provided. The cable arrangement system comprises the rotatable collimator, multiple supply cables for the collimator, the cables having a sufficient length in order to compensate for the rotation of the collimator and being arranged in at least two groups of supply cables, each group being arranged in such a way as to automatically adapt to a current rotational state of the collimator via at least one variable compensation loop section, the extent of the respective compensation loop section depending on the rotational state of the collimator, wherein the compensation loop sections of different groups are arranged separately from each other.

While traditional cable windup systems of radiotherapy devices are single-sided, the cable arrangement system according to the invention proposes two groups of supply cables. Hence the solution according to this invention can require less vertical space. Accordingly, the vertical profile of the whole assembly can be minimized. The concept according to this invention is therefore suitable to better avoid compromised cable management in tight environments. The subject-matter according to this invention allows to distribute cable height via the at least two groups, in particular to distribute the cable height evenly. Thus, space utilization can be optimized. Optionally, the number of groups may be adapted corresponding to the respective setup and design of the collimator. At the same time, cable organization and functionality can be maintained efficiently.

The term "rotatable collimator" is to be interpreted broadly. It generally describes a device that is capable of shaping, e.g. narrowing, a beam of particles or waves, in particular originating from a radiation source of the radiotherapy system. Furthermore, the collimator is rotatable. Hence, the collimator is configured to rotate about at least one rotational axis in at least one way. In particular, the collimator may be configured to rotate at least about a rotational axis in the center of the collimator and/or to rotate about itself and/or to rotate about a roll axis of the collimator. The rotational axis may in particular correspond to a vertical axis that is, in particular, approximately parallel to a general direction of the collimated radiation.

The multiple supply cables for the collimator may comprise any type of supply cables that are used to supply the collimator and/or that are needed at or around the collimator. The multiple supply cables may comprise at least two supply cables. For example, the supply cables may comprise at least one signal cable, at least one power cable, and/or at least one ground cable. Preferably, the at least one signal cable may be in a different group than the at least one power cable. Putting these two kinds of cables in different groups may improve security and/or ensure that the signal transported via the signal cables is not disturbed by the electric current through the power cables. For example, the at least one signal cable may be or may comprise at least one ethernet cable. Preferably, there may be multiple signal cables, in particular in the form of multiple ethernet cables.

The cables, i.e., the supply cables, have a sufficient length in order to compensate for the rotation of the collimator. Hence the length of the cable is in particular configured such that, in their arrangement, they do not hinder the rotational movement of the collimator over the full extent of the rotatability of the collimator and/or that the cables are not strained due to insufficient length when the collimator is rotated. The cables are arranged in at least two groups of supply cables. In order to be able to adapt to a current rotational state of the collimator, each group of cables comprises at least one variable compensation loop section. The term "compensation loop section" is to be understood broadly. It means in particular that the cables in said section have at least one loop. The loop does not have to be a closed loop. In particular, the loop may be an open loop. The compensation loop sections or at least some of the compensation loop sections may be configured to make a turn, in particular at least approximately a U-turn, in particular a single turn. The U-turn may essentially be a 180-degree turn. For example, within the loop, the cables may roughly go in a first direction, be led around a bending section and go back in a direction that is about opposite to the first direction. The maximum extent of the loop section may be restricted by a blocking element, such as a bar, in particular a metal bar and/or a steel bar. Preferably, the bending has a maximum bend and/or a minimal radius of curvature. The loops of at least some of the groups, preferably of at least two groups and/or of all groups, may in particular be bent around the collimator such that their loop shape each follows the shape of the collimator. The compensation loop sections are arranged such that the extent of the respective compensation loop section depends on the rotational state of the collimator. In particular, the respective compensation loop may be configured such that the loop has a greater extent when the collimator is rotated in one direction, and has a smaller extent when the collimator is rotated in the other direction. Advantageously, the cables may thus be "stored" in the respective loop section of their group, when a lower cable length is required due to a current rotational state of the collimator. The compensation loop sections of different groups are arranged separately from each other. This means in particular, that the different groups are not part of the same compensation loop section. Hence, the cables may efficiently be distributed at different locations, for example at two sides of the collimator. This allows to reduce the amount of cables at a particular location and thus allows to reduce the required vertical space.

According to an embodiment, the loop section of a first one of the at least two groups extends in a first rotational direction around the collimator and the loop section of a second one of the at least two groups extends in a second rotational direction around the collimator opposite to the first rotational direction, such that the loops of the first and the second group extend at two different sides of the collimator, optionally at least mostly symmetrically, with respect to each other. Advantageously, the design of two groups being arranged in two rotational directions around the collimator may provide a particular space efficient arrangement of the cables that is, at the same time, particularly well-suited to protect the cables from wear due to the rotation of the collimator. The loops may extend mostly symmetrically, in particular being asymmetric by less than 10 percent, optionally being essentially symmetrically. A symmetric configuration may further ensure a balanced distribution of cable height and may further help to minimize the vertical profile of the arrangement. The loops of the first and second group are in particular bent around the collimator such that their loop shape each follows the shape of the collimator. The cables may be evenly distributed among the two groups. Additionally or alternatively, the cables may be distributed based on the design of the collimator, such that the available space around the collimator is evenly distributed among the cables. This may allow to further improve the reduction of vertical clearance.

According to an embodiment, the first group and the second group and/or all of the at least two groups each comprise essentially the same amount of supply cables in so far that the number of cables in both groups does not differ by more than two, preferably no more than one, cable(s) and/or in so far that a total cross section of the cables in any one group is not more than 50 percent greater than the total cross section of the cables in any other group. An arrangement of this embodiment may be a particularly easy and/or efficient way of enabling an evenly distribution of the cables.

According to an embodiment, a mechanism is provided that guides the supply cables in the loops of each group such that the supply cables are following a controlled path when the collimator is rotated. Advantageously, the mechanism may allow to increase the safety of the cables and allow to ensure that the cables do not move into a space they are not supposed to be in. Hence, it may be prevented that cables are crushed or block movable parts and/or the rotational movement of the collimator. For example, the mechanism may be or may be based on at least one cable chain.

According to an embodiment, a cable chain is provided for each of the groups that guides the supply cables of the respective group through the loop of the respective group. A cable chain may also be denoted as drag chain, cable carrier, or energy chain. It has been found that a cable chain is a particularly well-suited mechanism to lead the supply cables and ensure the safety of the supply cables and their controlled movement. A cable chain can ensure a smooth movement of the supply cables during collimator rotation, preventing tangling and reducing mechanical stress. The reduced stress on individual cables can improve durability and longevity of the cables. Advantageously, cable chains do not require much additional space, thus ensuring that vertical space vertical space constraints are not exceeded. The cable chains may be housed within enclosures that fit into the collimator structure.

According to an embodiment, the cable chains comprises solid links that are pivotably linked together and comprise an inner guide section to guide the supply cables. The links may be linked together such that a predetermined maximum bend between consecutive links is defined, in particular in each of two bent directions. Using solid links enables a particularly advantageous and flexible way of guiding the cables. In particular, a length of the cable chains may be easily adaptable, allowing an easier assembly of the arrangement.

According to an embodiment, the links are linked together such that the links are pivotable in two directions, namely a first direction corresponding to a bend of the loop of the respective group and a second direction which is opposite to the first direction. Preferably, the links are linked together such that a bending is only possible in the two opposite directions and is essentially prevented in any other direction. In this context "prevented in any other direction" is to be understood such, that there may still be a small amount of play in this other direction that is due to the design of the links. Advantageously, allowing to bend also in the second direction may allow to adapt the respective loop according to the shape of the collimator. Hence, the loop may be bent around the collimator.

According to an embodiment, the links are linked together such that a minimum possible radius of curvature of the cable chains in the first direction is set to be smaller than a minimum possible radius of curvature of the cable chains in the second direction. Advantageously this may enable a better control for guiding the supply cables. In particular, the minimum possible radius of curvature of the cable chains in the second direction may be adapted to correspond to an outer shape of the collimator and/or to an inner boundary the loop is led around. In particular, the minimum possible radius of curvature of the cable chains in the first direction may be set to be smaller than a minimum possible radius of curvature of the cable chains in the second direction.

According to an embodiment, the cable chains and/or the links of the cable chains are made from plastic material, in particular polyamide material, more specifically polyamide 6.6 material. Polyamide 6.6 material is also denoted as nylon material. Plastic material, in particular polyamide material, has been found to be particularly suitable for the cable chains in the context of this invention. Polyamide 6.6 material is advantageously both particularly suitably rigid to allow guidance for the cables as well as sufficiently light-weight and easy and cost-efficient to manufacture.

According to an embodiment, the cable chains are configured such that a minimum radius of curvature of the loop is restricted to a predefined radius. In particular, the minimum radius of curvature of the loop is restricted to larger than 75 mm. The restriction corresponds to a direction in which the cable is bendable, in particular in the first direction as described herein. Advantageously, such a minimum radius of curvature may allow to ensure that the supply cables are not bent more than the predefined bending, thus ensuring to avoid too much stress on the cables and to avoid impairing the cables' durability.

According to an embodiment, each cable chain is locally fixed at its ends via fixation means. Preferably, the respective cable chain is not fixed in between its ends. For example, the fixation means may comprise at least one screw and/or at least one bolt. Fixing the cable chains at their respective ends may enable an even better control and guidance over the supply cables.

According to an embodiment, an outward extend of the loop of each group is limited by an outer boundary, in particular a sheet metal outer boundary, and/or an inward extend of the loop of each group towards the collimator is limited by an inner boundary, in particular a sheet metal inner boundary. The outer boundary and/or the inner boundary may be of an essentially circular shape. The outer boundary may in particular be essentially concentric with the inner boundary. The inner boundary may correspond to the outer side of the collimator. Advantageously, providing an inner and/or outer boundary may provide a particularly efficient means to ensure a controlled guidance of the supply cables. The outer boundary and the inner boundary may allow a relative motion within the boundaries that is restricted and/or guided by the boundaries. In particular, the outer boundary and/or the inner boundary may be configured to control a motion of the respective cable chain.

According to an embodiment, the cables of each group are arranged above each other along a direction that is essentially parallel to the axis of rotation of the collimator. This may be a particularly efficient way of arranging the cables. The cables may be arranged partially above each and partially next to each other.

According to an embodiment, the loop of each group comprises a 180° turn, in particular a single 180° turn, the extent of the loop being in particular controlled by how far the 180° turn extends. A 180° turn is to be understood such that, after the turn in the loop, the cables are essentially led back in approximately parallel to the direction they are coming from.

According to an embodiment, at least two of the groups are arranged at essentially the same height in a direction parallel to the axis of rotation of the collimator. In particular, there may be exactly two groups that are at essentially the same height and/or all of the groups may be arranged at essentially the same height. This embodiment may in particular advantageous, with collimators that are of essentially symmetric design and/or that provide similar space for the two groups.

According to a further aspect of the invention, a radiotherapy device comprising the cable arrangement system as described herein is provided. All features and advantages of the cable arrangement system may be adapted to the radiotherapy device and vice versa.

According to an embodiment, the system is configured such that the loops of the supply cables can each be accessed from their respective side at the collimator. Therein, the system may in particular of modular design, that allows easy access to the cables. An individual access to the loops/groups of supply cables can facilitate repairs and/or replacements.

The embodiments described herein may be combined with each other unless indicated otherwise.

The accompanying drawings illustrate various exemplary embodiments and methods of various aspects of the invention.
Fig. 1 shows a rotatable collimator of a cable arrangement system for a radiotherapy device according to an embodiment of the invention;
Fig. 2 shows a section of an exemplary cable chain with corresponding links within which the supply cables are guided; and
Fig. 3 shows a radiotherapy device according to an embodiment of the invention.

Similar elements are designated with the same reference signs in the drawings.

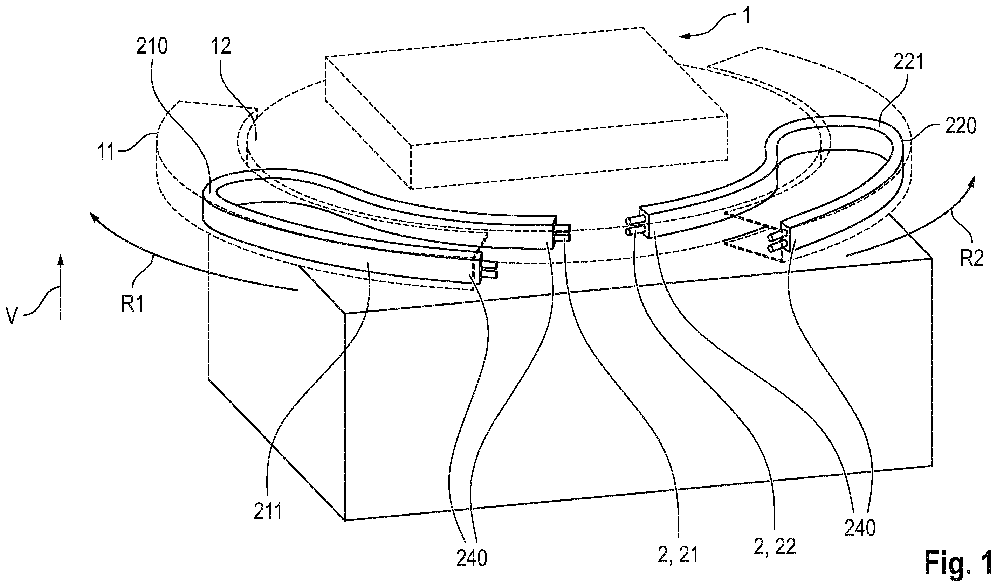

Figure 1 shows a rotatable collimator 1 of a cable arrangement system for a radiotherapy device 10 according to an embodiment of the invention. The cable arrangement system comprises the collimator 1 and multiple supply cables 2 for the collimator 1 that are arranged in two groups 21, 22. The cables 2 shown here, come from a gantry frame and are led towards a circuitry at the collimator 1. The cables 2 of each group 21, 22 are arranged above each other along a vertical direction V that is essentially parallel to the axis of rotation of the collimator and the two groups are arranged at essentially the same height in in this direction V. Each group of the two groups 210, 220 is arranged in such a way that it can automatically adapt to a current rotational state of the collimator 1. This is achieved via variable compensation loop sections 210, 220, the extent of which depends of the rotational state of the collimator 1. The loop 210, 220 of each group 21, 22 comprises a single 180° turn. Due to the loop sections 210, 220, the supply cables have a sufficient length in order to compensate for the rotation of the collimator 1. The extent of the loops 210, 220 is controlled by how far the respective 180° turn extends. The loop section 210 of the first group 21 extends in a first rotational direction R1 around the collimator 1 and the loop section 220 of the second groups 22 extends in a second rotational direction R2 around the collimator 1 opposite to the first rotational direction, such that the loops 210, 220 of the first group 21 and the second group 22 extend essentially symmetrically at two different sides of the collimator 1 and separately from each other. Furthermore, the loops 210, 220 of the first group 21 and second group 22 are bent around the collimator such that their loop shape each follows the shape of the collimator 1. Preferably, the first group 21 and the second group 22 may each comprise essentially the same amount of supply cables 2. Hence, the number of cables 2 in both groups 21, 22 may be arranged such that it does not differ by more than two, preferably no more than one, cable(s) 2 and/or in so far that a total cross section of the cables 2 in any one group is not more than 50 percent greater than the total cross section of the cables 2 in the other group. In this embodiment, a mechanism in the form of two cable chains 211, 221 is provided that guides the supply cables 2 in the loops 221, 220 of each group 21, 22 such that the supply cables 2 are following a controlled path when the collimator 1 is rotated. Preferably, each cable chain 210, 220 is locally fixed at its ends 240 via fixation means, such as screws. An outward extend of the loop 210, 221 of each group 21, 22 is limited by an outer boundary 11, in particular a sheet metal outer boundary, and an inward extend of the loop of each group towards the collimator is limited by an inner boundary 12, in particular a sheet metal inner boundary. Preferably, the cable chains 211, 221 may comprise solid links 230 that are pivotably linked together and comprise an inner guide section to guide the supply cables 2.

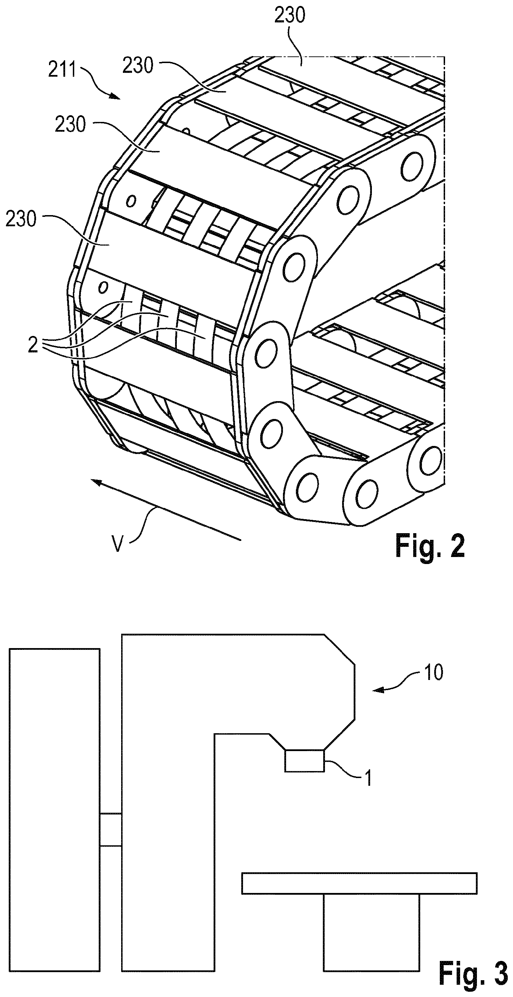

Figure 2 shows a section of an exemplary cable chain 211 with corresponding links 230 within which the supply cables are guided. In the section shown in figure 2 the cable chain 211 is bent in a first direction that corresponds to a bend of the loop 211 of the respective group 21. The cable chains 211, 221 are configured such that a minimum radius of curvature of the respective loop 210, 220, corresponding to the first direction, is restricted to a predefined radius. Preferably the links 230 are linked together such that the links 230 are pivotable in two directions, namely the first direction corresponding to a bend of the loop of the respective group and a second direction which is opposite to the first direction. This second direction corresponds to the slight bent of the loops 210, 220 around the collimator 1 as shown in figure 1. Preferably the links 230 are linked together such that a minimum possible radius of curvature of the cable chains 210, 220 in the first direction is set to be smaller than a minimum possible radius of curvature of the cable chains 210, 220 in the second direction that follows the curvature of the collimator 1.

Figure 3 shows a radiotherapy device 10 according to an embodiment of the invention. The radiotherapy device comprises a cable arrangement system according and a collimator 1 according to the invention. In this figure the, cable arrangement system around the collimator 1 is not shown in detail. It may, for example be the cable arrangement system as shown in figure 1.

### List of Reference

- 1: collimator
- 2: supply cables
- 10: radiotherapy device
- 11: outer boundary
- 12: inner boundary
- 21: first group of supply cables
- 22: second group of supply cables
- 210: first compensation loop section
- 211: cable chain of the first group
- 220: second compensation loop section
- 221: cable chain of the second group
- 230: cable chain links
- 240: ends of the cable chains
- R1: first rotational direction
- R2: second rotational direction
- V: vertical direction

## Claims

1. A cable arrangement system for a radiotherapy device (10) with a rotatable collimator (1), the cable arrangement system comprising
the rotatable collimator (1), and
multiple supply cables (2) for the collimator (1), the cables having a sufficient length in order to compensate for the rotation of the collimator and being arranged in at least two groups (21, 22) of supply cables (2), each group (21, 22,) being arranged in such a way as to automatically adapt to a current rotational state of the collimator (1) via at least one variable compensation loop section (210, 220), the extent of the respective compensation loop section (210, 220) depending on the rotational state of the collimator (1),
wherein the compensation loop sections (210, 220) of different groups (21, 22) are arranged separately from each other.

2. The cable arrangement system according to claim 1,
wherein the loop section (210) of a first one of the at least two groups (21, 22) extends in a first rotational direction (R1) around the collimator (1) and the loop section (220) of a second one of the at least two groups (21, 22) extends in a second rotational direction (R2) around the collimator (1) opposite to the first rotational direction (R1), such that the loops of the first and the second group (21, 22) extend at two different sides of the collimator (1) with respect to each other,
wherein the loops of the first and second group (21, 22) are in particular bent around the collimator (1) such that their loop shape each follows the shape of the collimator (1).

3. The cable arrangement system according to any one of the preceding claims,
wherein the first group (21) and the second group (22) each comprise essentially the same amount of supply cables (2) in so far that the number of cables in both groups (21, 22) does not differ by more than two, preferably no more than one, cable(s) and/or in so far that a total cross section of the cables in any one group (21, 22) is not more than 50 percent greater than the total cross section of the cables in any other group (21, 22).

4. The cable arrangement system according to any one of the preceding claims,
wherein a mechanism is provided that guides the supply cables (2) in the loops of each group (21, 22) such that the supply cables (2) are following a controlled path when the collimator (1) is rotated.

5. The cable arrangement system according to any one of the preceding claims,
wherein a cable chain (211, 221) is provided for each of the groups (21, 22) that guides the supply cables (2) of the respective group (21, 22) through the loop of the respective group (21, 22).

6. The cable arrangement system according to claim 5,
wherein the cable chains (211, 221) comprise solid links (230) that are pivotably linked together and comprise an inner guide section to guide the supply cables (2).

7. The cable arrangement system according to claim 5 or 6,
wherein the links (230) are linked together such that the links (230) are pivotable in two directions, namely a first direction corresponding to a bend of the loop of the respective group (21, 22) and a second direction which is opposite to the first direction.

8. The cable arrangement system according to claim 7,
wherein the links (230) are linked together such that a minimum possible radius of curvature of the cable chains (211, 221) in the first direction is set to be smaller than a minimum possible radius of curvature of the cable chains (211, 221) in the second direction.

9. The cable arrangement system according to any one of claims 5 to 8,
wherein the cable chains (211, 221) are configured such that a minimum radius of curvature of the respective loop, in particular in the first direction, is restricted to a predefined radius, in particular to be larger than 75 mm.

10. The cable arrangement system according to any one of claims 5 to 9,
wherein each cable chain (211, 221) is locally fixed at its ends (240) via fixation means, wherein the respective each cable chain (211, 221) is preferably not fixed in between its ends (240).

11. The cable arrangement system according to any one of the preceding claims,
wherein an outward extend of the loop of each group (21, 22) is limited by an outer boundary (11), in particular a sheet metal outer boundary, and/or
wherein an inward extend of the loop of each group (21, 22) towards the collimator (1) is limited by an inner boundary (12), in particular a sheet metal inner boundary.

12. The cable arrangement system according to any one of the preceding claims,
wherein the cables of each group (21, 22) are arranged above each other along a direction (V) that is essentially parallel to the axis of rotation of the collimator (1).

13. The cable arrangement system according to any one of the preceding claims,
wherein the loop of each group (21, 22) comprises a 180° turn, in particular a single 180° turn, the extent of the loop being in particular controlled by how far the 180° turn extends.

14. The cable arrangement system according to any one of the preceding claims,
wherein at least two of the groups (21, 22) are arranged at essentially the same height in a direction (V) parallel to the axis of rotation of the collimator (1).

15. A radiotherapy device (10) comprising the cable arrangement system according to any one of the preceding claims.
